# EUROPEAN PATENT SPECIFICATION

(11) **EP 4 486 216 B1**
(45) Date of publication and mention of the grant of the patent: **21.05.2025**
(21) Application number: 24717135.8
(22) Date of filing: 28.03.2024
(51) Int. Cl.: A61B 8/06, A61B 8/00, A61B 8/08

(54) **AN ULTRASOUND TRANSDUCER ATTACHMENT SYSTEM**
BEFESTIGUNGSSYSTEM FÜR ULTRASCHALLWANDLER
SYSTÈME DE FIXATION DE TRANSDUCTEUR À ULTRASONS

(30) Priority: 30.03.2023 GB 202304759
(43) Date of publication of application: 08.01.2025
(73) Proprietor: Cimon Medical AS, 7010 Trondheim (NO)
(72) Inventor: INGUL, Charlotte Björk, 7048 Trondheim (NO); HERGUM, Torbjørn, 5209 Os (NO); TORP, Hans, 7071 Trondheim (NO); HAGEN, Gard, 7014 Trondheim (NO)
(74) Representative: Dehns
(86) International application number: PCT/EP2024/058678
(87) International publication number: WO 2024/200766

(56) References cited:
- US-A1- 2012 197 118
- US-A1- 2012 277 597
- US-A1- 2015 045 675
- US-A1- 2018 353 157
- US-A1- 2019 388 056

## Description

### BACKGROUND OF THE INVENTION

This invention relates to an ultrasound transducer attachment system.

It is known to monitor blood flow in patients using an ultrasound transducer, for example using Doppler ultrasound techniques. US2019/0388056A1 discloses a standalone continuous cardiac Doppler or acoustic pulse monitoring patch. US2012/0197118A1 discloses an ultrasonic monitoring device for measuring physiological parameters of a mammal, the device including ultrasonic transducer elements coupled to a substrate. US2015/0045675A1 discloses a percutaneous device for carotid body ablation in which the percutaneous device may comprise an imaging modality such as an ultrasound transducer. US2012/0277597A1 discloses an apparatus for non-invasively assessing one or more physiologic parameters associated with a living organism.

It is also known that placing an ultrasound transducer accurately over a particular blood vessel, for diagnostic purposes, can be challenging, particularly where blood flow in a patient is weak or there is no blood flow. It is therefore desirable to provide an improved system for attachment of an ultrasound transducer adjacent a blood vessel.

The present invention seeks to provide an improved ultrasound transducer attachment system.

### SUMMARY OF THE INVENTION

From a first aspect, the invention provides an ultrasound transducer attachment system, comprising:
a substrate layer, having a first face and a second face, wherein the second face is on an opposite side to the first face, the first face comprising a first adhesive surface for bonding to skin on the neck of a human or animal;
an ultrasound transducer, fastened to the first face of the substrate layer, for placement adjacent a carotid artery of the patient; and
a removable alignment layer, removably bonded to the second face of the substrate layer and extending beyond the substrate layer in at least a first direction, along a first dimension, so as to aid alignment of the ultrasound transducer adjacent the carotid artery of the patient.

From a second aspect, the invention provides a method of attaching an ultrasound transducer to skin on the neck of a human or animal patient, aligned adjacent a carotid artery of the patient, wherein:
the ultrasound transducer is fastened to a first face of a substrate layer;
the first face comprises a first adhesive surface for bonding to the skin of the patient; and
a removable alignment layer is removably bonded to a second face of the substrate layer, on an opposite side to the first face, and extends beyond the substrate layer in at least a first direction, along a first dimension, so as to aid alignment of the ultrasound transducer adjacent the carotid artery of the patient,
the method comprising:
using the alignment layer to align the ultrasound transducer over the carotid artery of the patient;
applying pressure to the substrate layer to bond the first adhesive surface to the skin of the patient; and
removing the alignment layer from the substrate layer, thereby exposing a region of the neck of the patient.

Thus it will be seen that, in accordance with embodiments of the invention, a removable alignment layer extends beyond the substrate layer in such a way as to aid alignment of the ultrasound transducer with the carotid artery, thereby enabling an operator of the ultrasound transducer attachment system to more easily attach the ultrasound transducer to the patient in the desired position, with the ultrasound transducer adjacent the carotid artery of the patient (i.e. placed on the skin of the patient's neck in a position that is sufficiently close to and in line with the carotid artery to collect medically useful blood-flow data from the carotid artery). Moreover, since the alignment layer is removable from the substrate layer, it is able to be removed after placement of the ultrasound transducer so that less of the patient's neck is obscured by the system once the transducer is attached. In particular, at least in some embodiments, after removal of the removable alignment layer from the attachment system, only a portion of the patient's neck beneath the substrate layer is covered, rather than an entire area beneath the alignment layer (which extends beyond the substrate layer) being covered. The larger size of the alignment layer, relative to the substrate layer, helps the transducer to be placed more easily and accurately, but subsequent removal of this alignment layer still allows this alignment advantage to be maintained whilst also leaving more of the patient's neck exposed, e.g. for greater patient comfort and/or for ease of additional medical interventions.

The first adhesive surface may be suitable for bonding to skin adjacent the front of the neck of a human or animal, sometimes referred to as the throat.

The region of the neck that is exposed by removing the alignment layer from the substrate layer may lie at least partly on an opposite side of the neck to the ultrasound transducer. The region may extend over a second carotid artery and/or other blood vessels of the patient on the opposite side.

In some embodiments, the removable alignment layer comprises at least one alignment feature. In some embodiments, the at least one alignment feature comprises a cut-out notch, and/or a protrusion, and/or a visible surface mark such as a printed straight line. Such an alignment feature may be located on or over or along a centerline of the removable alignment layer.

The first dimension may be a length dimension. A second dimension (e.g. a width dimension) may be perpendicular to the first dimension.

Using the alignment layer to align the ultrasound transducer over the carotid artery of the patient may comprise aligning an alignment feature of the alignment layer with the center of the neck of the patient (i.e. aligned with the central vertical axis, or sagittal plane, of the patient's body). In some embodiments, the at least one alignment feature indicates a center of the removable alignment layer in the first dimension for alignment adjacent (i.e. over and in line with) the center of the neck of the patient. This may be achieved, for example, by aligning an alignment feature indicating the center of the removable alignment layer with the laryngeal prominence of the thyroid cartilage. This use of symmetry for the alignment layer may enable particularly straightforward and reliable alignment (both laterally and rotationally), even for an untrained operator.

In some embodiments, the substrate layer is located entirely to one side of a centerline of the removable alignment layer in the first dimension, i.e. such that the substrate layer is wholly located within a first half extent of the removable alignment layer in the first dimension. In some embodiments, at least a part of the substrate layer is located at or proximate to one end of the removable alignment layer in the first dimension.

In some embodiments, the removable alignment layer has an extent (e.g. a greatest extent, if it has more than one) in the first dimension that is double or at least double an extent (e.g. a greatest extent) of the substrate layer in the first dimension. The removable alignment layer may extend beyond the substrate layer in the first direction by a distance at least equal to this extent of the substrate layer in the first dimension. It may extend beyond the substrate layer in a direction opposite to the first direction (when the removable alignment layer is not folded), by less than this extent of the substrate layer in the first dimension.

In some embodiments, the removable alignment layer is elongate in the first dimension. In some embodiments, the removable alignment layer is symmetrical in the first dimension about a centerline. In some such embodiments, the substrate is also elongate in the first dimension.

The alignment layer may have a pair of parallel edges parallel to the first dimension. These may be joined by a pair of straight or curved edges. The substrate may be rectangular, and may be longer in the first dimension than in the second dimension.

In some embodiments, the removable alignment layer has an extent in the second dimension, perpendicular to the first dimension, that is the same as an extent of the substrate in the second dimension. In such embodiments, the removable alignment layer may extend beyond the substrate layer in the first dimension but not extend beyond the substrate layer in the second dimension, i.e. it extends sideways (lengthways) beyond the substrate layer, at least in the first direction, but not substantially upwards or downwards relative to the substrate layer. It will be appreciated that having the same extent may be exact but may allow for some margin of difference, e.g. of up to 10% or 5%, or up to 5mm or 2mm.

However, in other embodiments, the substrate layer extends further in the second dimension than the removable alignment layer (i.e., the removable alignment layer is narrower than the substrate layer). In yet other embodiments, the removable alignment layer extends further in the second dimension than the substrate layer (i.e., the removable alignment layer is wider than the substrate layer, such that it extends further than the substrate layer in both the first and second dimensions).

In some embodiments, the removable alignment layer has an extent in the first dimension of at least 200 mm, optionally of approximately 250 mm. In some embodiments, the removable alignment layer has an extent in the first dimension of less than 300 mm.

In some embodiments, the removable alignment layer extends beyond the substrate layer in the first direction by at least 100 mm, optionally approximately 125 mm. However, in some embodiments, the removable alignment layer extends beyond the substrate layer in the first direction by no more than 200 mm.

In some embodiments, the substrate layer has an extent in the first dimension of at least 50 mm or 100 mm, e.g. approximately 100 mm. However, in some embodiments, the substrate layer has an extent in the first dimension of no more than 125 mm (e.g. being confined to one half of removable alignment layer, when this is approximately 250 mm in length).

In some embodiments, the substrate layer and/or the removable alignment have an extent in the second dimension of approximately 50 mm.

In some embodiments, the substrate layer comprises a flexible sheet of plastics material. The flexible sheet of plastics material may comprise a layer of plastic foam, covered on one side with polypropylene and on the other side with polyethylene.

In some embodiments, the substrate layer comprises (or consists of) a hydrogel. The hydrogel may comprise (or consist of) an embedded mesh to make it easier to manufacture and handle. The hydrogel may be laminated, and may have a different hydrogel formulation on each side of the mesh. This may help to give the two sides different properties, for instance different tackiness. The substrate layer may further comprise a layer of (e.g. flexible) plastic foam, and/or one or more layers of adhesive (i.e. providing the second adhesive surface).

In some embodiments, the first adhesive surface comprises (e.g. is provided by) a hydrogel (i.e. the substrate layer comprises a hydrogel (layer), providing the first adhesive surface).

In some embodiments, the substrate layer comprises a (first) slit, which may be arranged to accommodate a cable of the ultrasound transducer within the slit and/or allow a cable of the ultrasound transducer to pass through the slit (i.e. travelling forwards). The (first) slit may extend along the first dimension. The (first) slit may be positioned adjacent to a point at which the ultrasound transducer is attached to the substrate layer.

In some embodiments, the removable alignment layer comprises a (second) slit, which may be arranged to accommodate a cable of the ultrasound transducer within the slit and/or allow a cable of the ultrasound transducer to pass through the slit (i.e. travelling forwards). The (second) slit may extend along the first dimension. It may be aligned over the first slit.

In some embodiments, the ultrasound transducer is offset in the first dimension from a centerline of the removable alignment layer. It may be offset from the centerline in a direction opposite to the first direction, when the removable alignment layer is not folded. In some embodiments, the ultrasound transducer is offset in the first dimension from a centerline of the removable alignment layer such that a center of the ultrasound transducer is approximately 50 mm from the centerline of the removable alignment layer in the first dimension.

In some embodiments, the ultrasound transducer attachment system further comprises a backing cover, covering the first adhesive surface of the substrate layer. The method may comprise removing the backing cover to expose the first adhesive surface. The ultrasound transducer may be positioned between the substrate layer and the backing cover. In some embodiments, the backing cover comprises a tab extending away from the substrate layer and/or the removable alignment layer, to aid removal of the backing cover from the substrate layer. The tab may extend in the first dimension. It may extend in a direction opposite to the first direction when the removable alignment layer is unfolded.

In some embodiments, the removable alignment layer comprises a first portion, removably bonded to the second face of the substrate layer, and a second portion, extending beyond the substrate layer in the first direction. The second portion may be bonded (e.g. non-removably) to the backing cover. The removable alignment layer may have a folded configuration in which the backing cover is covering the first adhesive surface of the substrate layer. Thus, in some embodiments, the method further comprises removing the backing cover to expose the first adhesive surface, such that as the backing cover is removed, the removable alignment layer is opened out from a folded configuration, e.g. to a substantially flat (or planar) configuration. It will be appreciated that in use, when subsequently conformed to the neck of the patient, the removable alignment layer will not be planar, but will curve around the neck. The second portion may be made of a non-adhesive material and may comprise an adhesive bonding material arranged between the second portion and the backing cover, to bond them together.

It will be appreciated that whether or not a part is bonded "non-removably" or "removably" refers to whether it will be removed by normal force applied by an operator, not whether there is any physical means which will enable removal. For example, where the second portion is non-removably bonded to the backing cover, this may simply require that the second portion is bonded sufficiently strongly that the second portion will not be removed from the backing layer as the backing layer is removed from the first adhesive surface. Thus, in some embodiments, the second portion is bonded to the backing cover with an adhesive strength that is greater than an adhesive strength between the backing cover and the first adhesive surface. Adhesive strength may be tensile and/or shear and/or peel strength, and may be determined in any appropriate way.

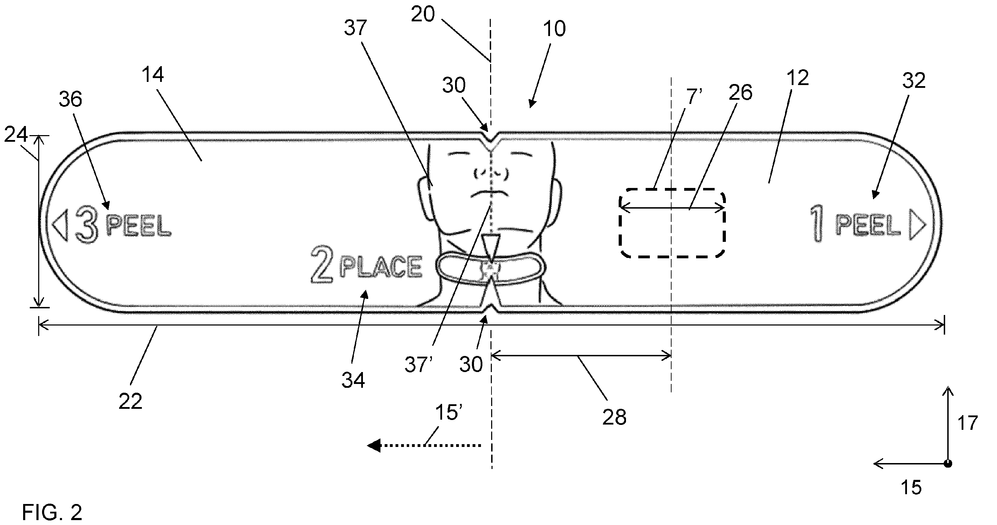

In other embodiments, the second portion may not be bonded to the backing cover (e.g. such that the removable alignment layer may always have an open configuration, as shown in Figure 2 which is discussed in greater detail below). The second portion may be made of a non-adhesive (e.g. paper-like) material and/or may have a non-adhesive surface.

Alternatively, in other embodiments, the removable alignment layer comprises a first portion, removably bonded to the second face of the substrate layer, and a second portion, extending beyond the substrate layer in the first direction and removably bonded to the first adhesive surface of the first face of the substrate layer. Thus, a portion of the backing cover that contacts the first face of the substrate layer (i.e. the face to which the transducer is fastened) may provide a backing cover for the first adhesive surface (i.e. when the removable layer is in a folded configuration). Thus, the method may further comprise removing the second portion from the first adhesive surface to expose the first adhesive surface. This has the advantage that no separate backing cover is required since the second portion of the removable alignment layer provides the backing cover.

In some embodiments, the ultrasound transducer attachment system further comprises an acoustic coupling layer positioned between the ultrasound transducer and the backing cover. For example, the acoustic coupling layer may be an ultrasound coupling gel, a hydrogel, or a silicone compound.

In some embodiments, the removable alignment layer is bonded to the substrate layer. The removable alignment layer may comprise a second adhesive surface, wherein the second adhesive surface is bonded to the substrate layer. The first portion of the removable alignment layer may comprise the second adhesive surface.

The first adhesive surface may extend across the entire length and/or the entire width of the first face of the substrate layer (e.g. across the entire first face), or it may extend across only part of the length and/or width of the first face. The first face may comprise a plurality of first adhesive surfaces (i.e. areas of adhesive, separated by non-adhesive surfaces). In some embodiments, an adhesive strength between the removable alignment layer and the substrate layer is less than an adhesive strength between the first adhesive surface of the substrate layer and the skin of the patient, once the first adhesive surface has been bonded to the skin, at least in typical usage. This can enable the removable alignment layer to be removed from the substrate layer without removing the substrate layer (i.e. from the skin of the patient) after the first adhesive surface is bonded to the patient. In some embodiments the first adhesive surface comprises a first adhesive and the second adhesive surface comprises a second adhesive, wherein the second adhesive is weaker than the first adhesive. However, the use of two different adhesives is not required in order to achieve different adhesive strengths for the different bonds. This could be achieved in any suitable manner. For example, in addition or alternatively to using two different adhesives, a smaller area (e.g. total aggregate area) of adhesive may be used on the first adhesive surface of the substrate layer, compared to the total (e.g. aggregate) area of adhesive used between (i.e. to bond) the alignment layer and the substrate layer.

It will be understood that the substrate layer may similarly (i.e. using any of the methods described above) be bonded more strongly to the alignment layer than to the backing cover, i.e. such that the substrate layer stays bonded to the alignment layer as the backing cover is removed.

In some embodiments, the removable alignment layer is flexible, for conforming the removable alignment layer to the neck of the patient. In some embodiments, the method comprises aligning the alignment layer such that the first direction lies perpendicular to a median plane of the patient (i.e. with the first direction extending around the neck of the patient, substantially parallel to a transverse plane of the patient). In some embodiments, the method comprises conforming the removable alignment layer to the neck of the patient before removing the alignment layer.

Approximately, as used herein, may mean to within +/-10% or +/-5% or to any other appropriate degree.

Features of any aspect or embodiment described herein may, wherever appropriate, be applied to any other aspect or embodiment described herein. Where reference is made to different embodiments or sets of embodiments, it should be understood that these are not necessarily distinct but may overlap.

### BRIEF DESCRIPTION OF THE DRAWINGS

Certain preferred embodiments of the invention will now be described, by way of example only, with reference to the accompanying drawings, in which:
Figure 1 is an exploded view showing components of an ultrasound transducer attachment system according to an embodiment of the present invention;
Figure 2 is a front view of the removable alignment layer of Figure 1;
Figure 3 is a perspective view of the ultrasound transducer attachment system of Figure 1, before being applied to a patient;
Figure 4 is a perspective view of the ultrasound transducer attachment system of Figure 1, in the process of being unfolded;
Figure 5 is a flow diagram representing steps of a method of attaching an ultrasound transducer to a patient, according to an embodiment of the present invention; and
Figure 6 is an exploded view showing components of an ultrasound transducer attachment system according to a second embodiment of the present invention.

### DETAILED DESCRIPTION

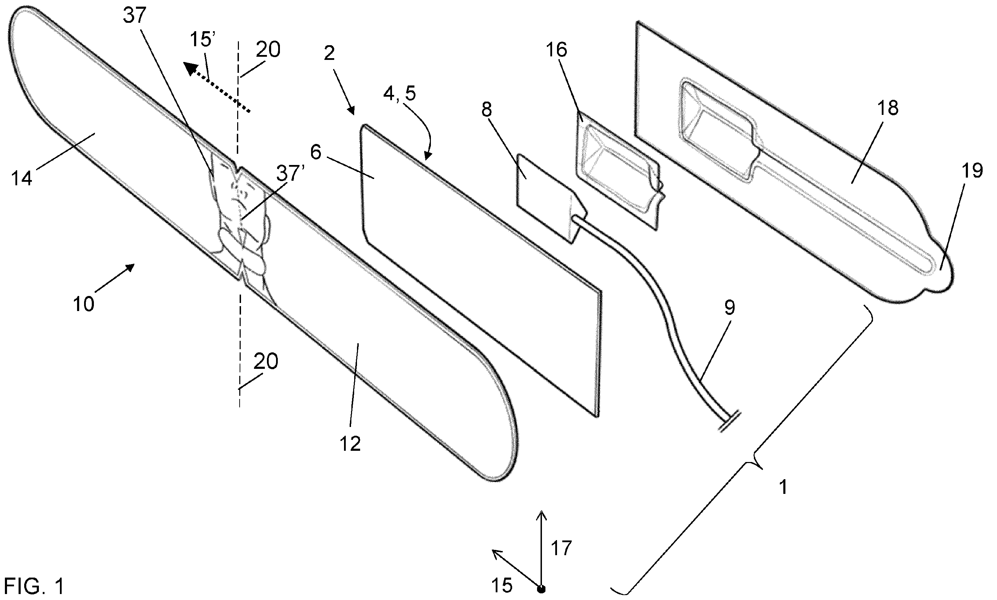

Figure 1 shows an ultrasound transducer attachment system 1. The ultrasound transducer attachment system 1 includes a substrate layer 2, which has a first, inward-facing face 4, and a second, outward-facing face 6. The outward-facing face 6 is visible from the view of Figure 1 and is on an opposite side to the inward-facing face 4. The substrate layer 2 is principally made of a flexible sheet of plastics material, with an adhesive coating layer 5 provided on the inward-facing face 4. The flexible sheet of plastics material may be a laminate of a layer of "plastic foam" (e.g. Foam 3M 1773), covered on one side (e.g. on the outward-facing face 6) with polypropylene (e.g. TC50/RC10) and on the other side with polyethylene. Alternatively, in other examples, instead of polyethylene the flexible sheet of plastics material may be covered on the other side with a hydrogel. The adhesive used for the adhesive coating layer 5 may be skin adhesive 3M 4075, or it may alternatively be a hydrogel such as Axelgaard AG2530. The Applicant has appreciated that use of a hydrogel adhesive may provide particularly effective bonding of the attachment system 1 to certain patients, for example those whose skin is damp and/or cold, as is common among patients suffering from a cardiac arrest. The inward-facing face 4 thus includes a first adhesive surface for bonding to the skin of a patient.

The ultrasound transducer attachment system 1 also includes an ultrasound transducer 8, which is fastened to the inward-facing face 4 of the substrate layer 2, for placement adjacent a carotid artery of the patient, as explained further below. Figure 1 shows the rear surface of the ultrasound transducer 8; the transducer 8 is arranged to emit ultrasound signals from, and receive reflected signals at, one or both of two angled faces that face away from the substrate layer 2 and towards the patient once the transducer 8 has been applied to the patient. A cable 9 extends from the ultrasound transducer 8, and can be connected to a separate device which may send electrical signals to, and receive and process electrical signals from, the ultrasound transducer 8. For example, the cable 9 may be connected (at its other end, not shown) to a resuscitation device, such as a defibrillator, or to a medical monitoring system.

The ultrasound transducer attachment system 1 also includes a removable alignment layer 10. This may be principally made of a flexible sheet of paper or plastics material, e.g. a 50 µm thick polyester sheet. The removable alignment layer 10 includes a first portion 12 that overlaps with (i.e. covers) the outward-facing face 6 of the substrate layer 2 and is removably bonded to it, e.g. by an adhesive coating to the removable alignment layer 10. The adhesive coating may be, for example, RP51. The removable alignment layer 10 also includes a second portion 14 that extends beyond the substrate layer 2 in a first direction 15', parallel to a first "length" dimension 15, by a substantial amount, so as to aid alignment of the ultrasound transducer 8 adjacent the carotid artery of the patient. In this example, the removable alignment layer 10 also extends beyond the substrate layer 2, in the opposite direction to the first direction 15' (when the layer 10 is unfolded), but by a much lesser amount.

The ultrasound transducer attachment system 1 further includes an acoustic coupling layer 16, which in this example is a hydrogel (e.g. hydrogel AG625) for acoustic impedance matching, and is arranged partially or wholly over at least one surface (i.e. the sound-emitting and/or sound-receiving face or faces) of the ultrasound transducer.

A backing cover 18, e.g. of a formed plastics material, is arranged as a removable cover over the acoustic coupling layer 16 and the ultrasound transducer 8. It also extends to removably cover the adhesive surface of the inward-facing face 4 of the substrate layer 2.

Figure 2 provides a more-detailed front view of the removable alignment layer 10, showing the outward-facing face of this layer 10 (i.e. the face not in contact with the outward-facing face 6 of the substrate layer 2). Various reference lines 7', 15, 15', 20, 17, 24, 26, 28 are shown in Figure 2 that are not part of the removable alignment layer 10 itself. However, the removable alignment layer 10 does carry a printed design on its outward surface, comprising three written elements 32, 34, 36 and a diagram 37, as described in more detail below.

It can be seen from Figure 2 that, in this instance, this first direction 15' is from right to left along the removable alignment layer 10.

The first portion 12 is located to the right side of the dashed line 20, whilst the second portion 14 is at least predominantly to the left side of the dashed line 20. The dashed line 20 represents a centerline, at a mid-point in the length dimension 15, of the removable alignment layer 10.

In this example, the removable alignment layer 10 has a greatest extent 22 in the length dimension 15 of approximately 250 mm. The removable alignment layer 10 has a greatest extent 24 in a second "width" dimension 17, perpendicular to the length dimension 15, of approximately 50 mm.

The substrate layer 6 (not visible in Figure 2) is rectangular in this example, with an extent in the length dimension 15 of approximately 100 mm and an extent in the width dimension 17 of approximately 48 mm.

The ultrasound transducer 8 cannot be seen from the viewpoint of Figure 2, but for reference its position is represented by a dashed rectangle 7'. The ultrasound transducer in this example has an extent 26 in the length dimension 15 of approximately 30 mm. It is offset to the right of the centerline 20 of the removable alignment layer 10 such that a center of the ultrasound transducer 7' is a distance 28 of approximately 50 mm from the centerline 20.

The centerline 20 of the removable alignment layer 10 is indicated by a pair of cut-out notches 30 which are positioned on the centerline 20, and also by a printed centerline 37' on the outward face of the alignment layer 10, coincident with the conceptual centerline 20. These provide alignment features which facilitate easy placement of the ultrasound transducer 8 over the carotid artery of a patient. In particular, a person using the ultrasound transducer attachment system 1 (e.g. a first-aid responder) need only align the vertical line 37' and notches 30 with the centerline of a patient's neck and throat area (i.e. to lie in a sagittal plane of the patient), to achieve good positioning of the ultrasound transducer over the patient's carotid artery. This may be achieved, for example, by aligning the printed centerline 37' and/or the notches 30 with the laryngeal prominence of the thyroid cartilage, where this is present in a patient. The dimensions of the transducer 8 and the lateral distance 28 are selected such that at least part of the transducer 8 is expected to overlie the carotid artery of any patient, regardless of sex, age or size, or at least a defined class of patients (e.g. for 90% of adults).

Figures 1 and 2 show the removable alignment layer 10 in a planar "opened out" configuration. However, it will be appreciated that, when applied to a patient, the removable alignment layer 10 will be curved to conform to the neck of the patient.

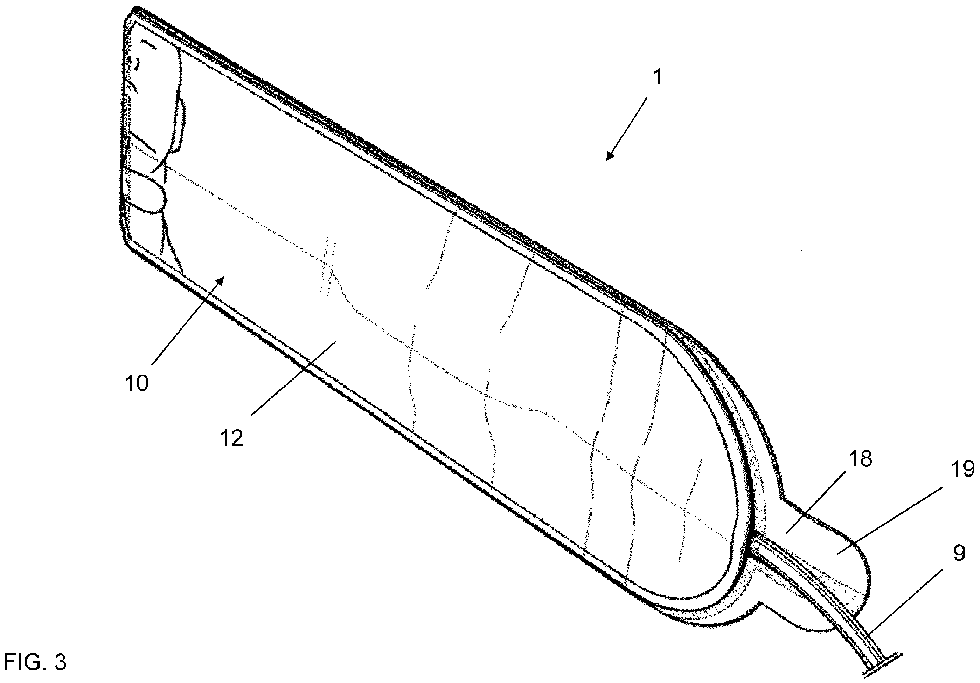

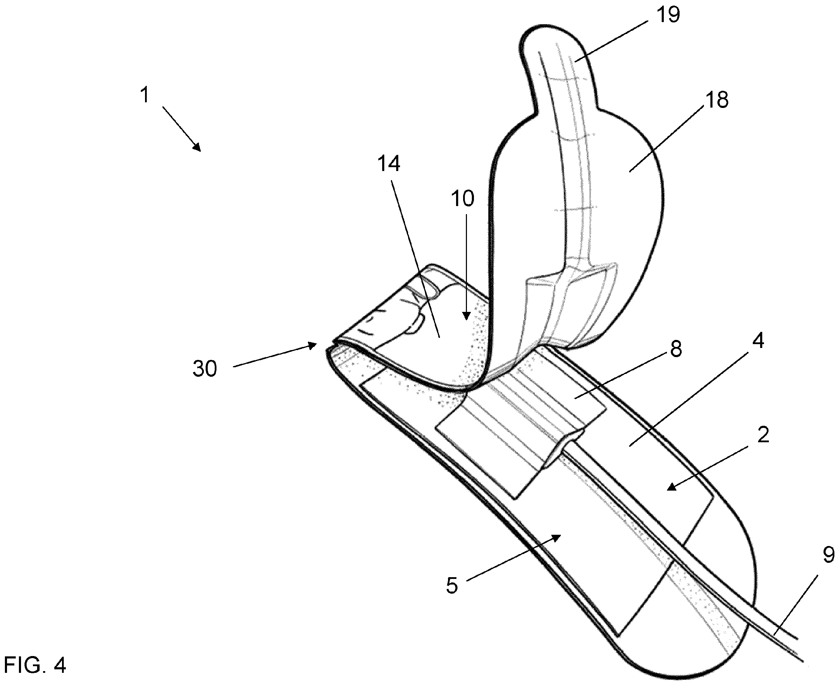

Furthermore, in some embodiments, in order to make the ultrasound transducer attachment system 1 more compact (e.g. for transport before application to a patient) and to improve operator convenience, the removable alignment layer 10 may be folded in half about the centerline 20 for storage, prior to use, such that the second portion 14 of the removable alignment layer 10 lies adjacent the first portion 12. The second portion 14 may be bonded to the backing cover 18. This storage configuration is illustrated in Figures 3 and 4.

The textual graphical elements 32, 34, 36 shown in Figure 2 have been omitted from Figures 3 and 4 for simplicity. As shown in Figure 3 and 4, for some embodiments the removable alignment layer 10 begins in a folded configuration, in which the first portion 12 is bonded to the inward-facing face 6 of the substrate layer 2 by a first adhesive strength (or bonding force), whilst the second portion 14 is bonded to a rear surface of the backing cover 18 by a second adhesive strength (or bonding force), wherein the second adhesive strength is stronger than the first adhesive strength (wherein the rear surface faces away from the coupling layer 16). The bonding between the first portion 12 and the inward-facing face 6 and/or between the second portion 14 and the rear surface of the backing cover 18 may be provided by adhesive RP51 (402-034-A). It will be appreciated that differing bonding forces may be achieved even where the same adhesive is used in both bonds, by varying a configuration of the adhesive, such as by providing a greater area of adhesive for one of the bonds than the other.

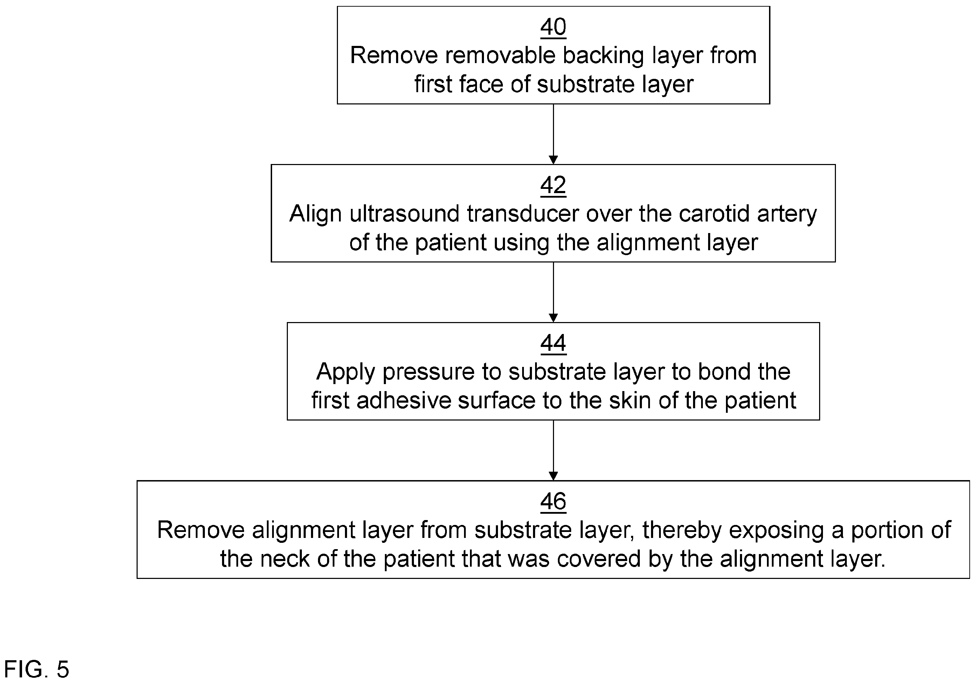

Figure 5 is a flow diagram showing steps of applying the ultrasound transducer attachment system 1 to attach a transducer element 8 onto the skin of a patient overlying the carotid artery.

Three of these steps are represented by the printed graphical elements 32, 34, 36 on the removable alignment layer 10 (see Figure 2), in order to provide an operator of the ultrasound transducer attachment system with clear guidance and instruction on how to carry out attachment of the ultrasound transducer. The steps are summarised by the printed words "PEEL" 32, "PLACE" 34 and "PEEL" 36. In the illustrated example these steps are also numbered to help an operator carry out these steps in the preferred order.

For some embodiments, the attachment system 1 is initially folded for storage, as shown in Figure 3.

At a first step, 40, of the application process, the backing cover 18 is separated from the inward-facing face 4 of the substrate layer 2 by pulling on a protruding tab 19 of the backing cover 18, as illustrated in Figure 4. This exposes an adhesive coating 5 of the inward-facing face 4, and also exposes the acoustic coupling layer 16. In doing this, the second portion 14 of the removable alignment layer 10 is opened out into an open position, giving the removable alignment layer 10 substantially the same configuration as that shown in Figures 1 and 2. This step is represented by the graphical element "PEEL" 32 in Figure 2 which provides an operator with the instruction to peel the backing cover 18 away first.

Next, at step 42, the operator aligns the printed centerline 37' and notches 30 with the centerline of the patient's neck, and applies sufficient pressure to the removable alignment layer 10 to conform it to the patient's neck. This results in the ultrasound transducer being aligned over the carotid artery of the patient, allowing useful data (e.g. Doppler blood flow data) relating to the carotid artery of the patient to be collected using the ultrasound transducer 8. An active face of the transducer 8 is angled, so as to direct ultrasound signals non-perpendicularly to the carotid artery, since only the velocity-component perpendicular to the transducer face can be measured accurately using Doppler ultrasound. This step is represented by the graphical element "PLACE" 34, instructing the operator to "place" the ultrasound transducer attachment system 1 in position, and further including an image 37 of a person's head and neck representing the position in which the system 1 is to be placed.

Then, at step 44, the operator applies firmer pressure to the substrate layer 2, for example by pressing down harder on the right portion 12 of the removable alignment layer 10, to bond the adhesive coating 5 of the inward-facing face 4 of the substrate layer 2 onto the skin of the patient's neck, so that the ultrasound transducer 8 is secured in place by the adhesive 5 and by tension in the substrate layer 2 and cannot easily move out of position, e.g. even if the cable 9 is tugged.

Finally, at step 46, the operator of the ultrasound transducer attachment system 1 removes the removable alignment layer 10 from the substrate layer 2, by peeling from the end indicated by the third graphical element "PEEL" 36. This exposes a region of the neck of the patient that would otherwise have been covered by the removable alignment layer 10-in particular, the region that was previously located under the second portion 14, leaving this side of the patient's neck, opposite from the ultrasound transducer 8, available and exposed for other medical interventions.

It will be appreciated that, since many animals, including humans, have a carotid artery on each side of their neck, the ultrasound transducer attachment system 1 may be used either way up, i.e. in the orientation shown in Figure 2, or rotated through 180 degrees in the plane of the page of Figure 2. Thus the ultrasound transducer 8 may be positioned on the right side or left side of the patient's neck, depending on the orientation of the ultrasound transducer attachment system 1. Studies suggest that approximately 90% of people are right-handed and the graphics shown on the patch are therefore arranged to illustrate, and primarily aid with, placement of the ultrasound transducer on the patient's left side (from the perspective of the patient), as this is easier for a right-handed operator. This configuration leaves the right side of the patient's neck free for venous access. Left-handed operators may rotate the patch 180 degrees before application, and will still obtain useful information from the graphical elements to aid with this process even though they are upside-down.

Providing an alignment layer 10 that extends substantially beyond the substrate layer 2 (e.g. being at least twice as long as the substrate layer 2 in some embodiments), enables easy and accurate symmetrical placement of the alignment layer 10 with the ultrasound transducer 8 in a position aligned over (i.e. aligned adjacent) a carotid artery of the patient. Moreover, the alignment layer 10 is removable such that, once it has fulfilled the function of aiding transducer placement on a patient's neck, it can be removed from the substrate layer 2 so as to expose more of the patient's neck than would be the case if the alignment layer 10 remained in place. After removal, only the region of the patient's neck that is beneath the substrate layer 2 is covered, but the rest of the neck is uncovered and accessible for other medical interventions (e.g. during a cardiac arrest the internal jugular vein is sometimes used for venous access). This ultrasound transducer attachment system 1 therefore minimises the obstruction of a patient's neck once the transducer 8 has been fixed in place, whilst facilitating easy and accurate placement of the ultrasound transducer 8 in the first place.

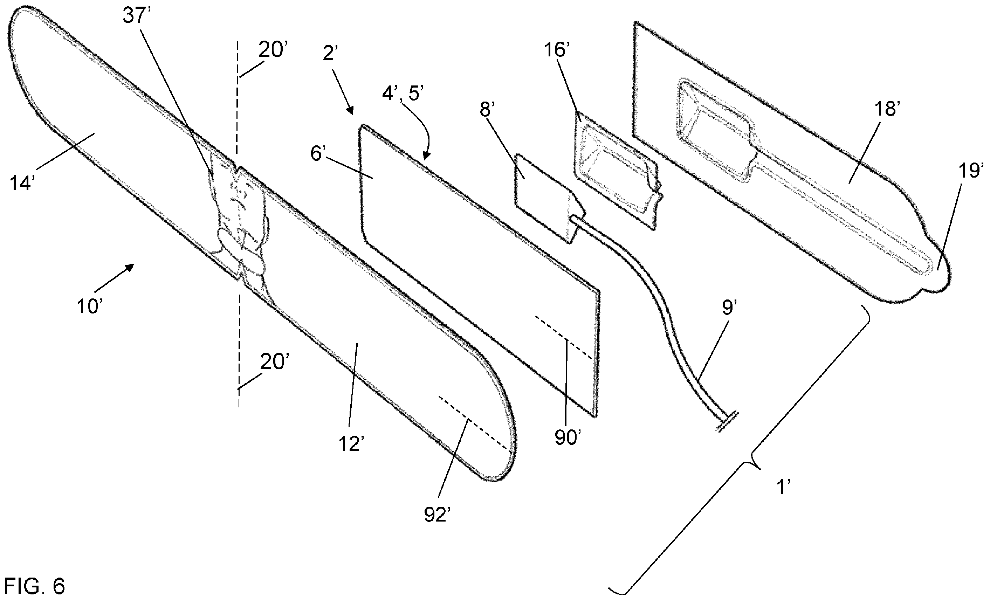

Figure 6 shows a transducer attachment system 1' according to a second embodiment of the present invention. It includes all the same components as the embodiment of Figure 1. For convenience these are all labelled using the same reference numerals as are used in Figure 1, but in the case of Figure 6 these reference numerals are now followed by an apostrophe.

In this embodiment, a slit is formed in certain components of the transducer attachment system 1', allowing the cable 9' to exit the transducer attachment system 1' away from the ultrasound transducer 8'. These slits are represented in Figure 6 by dashed lines.

A first slit 90' is formed in the substrate layer 2', extending from an outer edge of the substrate layer 2' (the right-hand edge in the view of Figure 6) inwards along the first dimension. This allows the cable 9' to move further away from the ultrasound transducer 8' as it exits the attachment system 1', since it can move at least in line with the substrate layer 2' by being accommodated within the slit 90'.

A second slit 92' is formed in the removable alignment layer 10' in this example, and is aligned with the first slit 90'. It extends inwards from an outer edge of the removable alignment layer 10', along the first dimension. This allows the cable 9' to move even further away from the ultrasound transducer 8' along its length, and optionally allows it to extend in a forward direction (i.e. substantially perpendicular to the face of the ultrasound transducer 8') if desired. In other embodiments, this second slit 92' need not be present and instead the cable 9' may extend between the substrate layer 6' and the removable alignment layer 10'.

The Applicant has appreciated that the presence of the slit or slits may improve adhesion of the ultrasound transducer 8' to a patient, and/or reduce a risk of dislodging the ultrasound transducer 8' in the event that a pulling force is accidentally applied to the cable 9, since the force will transfer to the attachment system 1' closer to the ultrasound transducer 8' rather than at an outer edge of the substrate layer 2'.

It will be appreciated by those skilled in the art that the invention has been illustrated by describing one or more specific embodiments thereof, but is not limited to these embodiments; many variations and modifications are possible, within the scope of the accompanying claims.

## Claims

1. An ultrasound transducer attachment system (1, 1'), comprising:
a substrate layer (2, 2'), having a first face (4, 4') and a second face (6, 6'), wherein the second face (6, 6') is on an opposite side to the first face (4, 4'), the first face (4, 4') comprising a first adhesive surface (5, 5') for bonding to skin on the neck of a human or animal patient;
an ultrasound transducer (8, 8'), fastened to the first face (4, 4') of the substrate layer (2, 2'), for placement adjacent a carotid artery of the patient; and
**characterised by** further comprising a removable alignment layer (10, 10'), removably bonded to the second face (6, 6') of the substrate layer (2, 2') and extending beyond the substrate layer (2, 2') in at least a first direction (15'), along a first dimension, so as to aid alignment of the ultrasound transducer (8, 8') adjacent the carotid artery of the patient.

2. The ultrasound transducer attachment system (1, 1') of claim 1, wherein the removable alignment layer (10, 10') comprises at least one alignment feature (37'); optionally
wherein the at least one alignment feature (37') comprises a cut-out notch, and/or a protrusion, and/or a visible surface mark; and/or
wherein the at least one alignment feature (37') indicates a center of the removable alignment layer (10, 10') in the first dimension for alignment adjacent a center of the neck of the patient.

3. The ultrasound transducer attachment system (1, 1') of any preceding claim, wherein the substrate layer (2, 2') is located entirely to one side of a centerline of the removable alignment layer (10, 10') in the first dimension; and/or
wherein the substrate layer (2, 2') has an extent in the first direction of at least 50 mm and of at most 125 mm.

4. The ultrasound transducer attachment system (1, 1') of any preceding claim, wherein the removable alignment layer (10, 10') has a greatest extent in the first dimension that is at least double a greatest extent of the substrate layer (2, 2') in the first dimension; and/or
wherein the removable alignment layer (10, 10') is elongate in the first dimension; and/or
wherein the removable alignment layer (10, 10') has an extent in the first dimension of at least 200 mm.

5. The ultrasound transducer attachment system (1, 1') of any preceding claim, wherein the removable alignment layer (10, 10') has an extent in a second dimension, perpendicular to the first dimension, that is the same as an extent of the substrate layer (2, 2') in the second dimension.

6. The ultrasound transducer attachment system (1, 1') of any preceding claim, wherein the removable alignment layer (10, 10') extends beyond the substrate layer (2, 2') in the first direction by at least 100 mm.

7. The ultrasound transducer attachment system (1, 1') of any preceding claim, wherein the ultrasound transducer (8, 8') is offset from a centerline of the removable alignment layer (10, 10') in a direction opposite to the first direction; optionally
wherein the ultrasound transducer (8, 8') is offset from the centerline of the removable alignment layer (10, 10') such that a center of the ultrasound transducer (8, 8') is approximately 50 mm from the centerline of the removable alignment layer (10, 10') in the first dimension.

8. The ultrasound transducer attachment system (1, 1') of any preceding claim, further comprising a backing cover (18, 18'), covering the first adhesive surface (5, 5') of the substrate layer (2, 2'), wherein the ultrasound transducer (8, 8') is positioned between the substrate layer (2, 2') and the backing cover (18, 18'); optionally
wherein the removable alignment layer (10, 10') comprises a first portion (12, 12'), bonded to the second face (6, 6') of the substrate layer (2, 2') with a first adhesive strength, and a second portion (14, 14'), extending beyond the substrate layer (2, 2') in the first direction, and bonded to the backing cover (18, 18') with a second adhesive strength greater than the first adhesive strength; and/or
wherein the backing cover (18, 18') comprises a tab (19, 19') extending away from the substrate layer (2, 2') in the first dimension, to aid removal of the backing cover (18, 18') from the substrate layer (2, 2').

9. The ultrasound transducer attachment system (1, 1') of claim 8, further comprising an acoustic coupling layer (16, 16') positioned between the ultrasound transducer (8, 8') and the backing cover (18, 18'); optionally
wherein the acoustic coupling layer (16, 16') is an ultrasound coupling gel, a hydrogel, or a silicone compound.

10. The ultrasound transducer attachment system (1, 1') of any preceding claim, wherein the removable alignment layer (10, 10') is bonded to the substrate layer (2, 2') with an adhesive strength that is less than an adhesive strength between the first adhesive surface of the substrate layer and the skin of the patient, once the first adhesive surface is applied to the patient.

11. The ultrasound transducer attachment system (1, 1') of any preceding claim, wherein the first adhesive surface (5, 5') comprises a hydrogel.

12. The ultrasound transducer attachment system (1') of any preceding claim, wherein the substrate layer (2') comprises a slit (90') arranged to accommodate a cable (9') of the ultrasound transducer (8') within the slit (90') and/or to allow the cable (9') to pass through the slit (90'); and/or
wherein the removable alignment layer (10') comprises a slit (92') arranged to accommodate a cable (9') of the ultrasound transducer (8') within the slit (92') and/or to allow the cable (9') to pass through the slit (92').

13. A method of attaching an ultrasound transducer (8, 8') to skin on the neck of a human or animal patient, aligned adjacent a carotid artery of the patient, wherein:
the ultrasound transducer (8, 8') is fastened to a first face (4, 4') of a substrate layer (2, 2');
the first face (4, 4') comprises a first adhesive surface (5, 5') for bonding to the skin of the patient; and
**characterised in that** a removable alignment layer (10, 10') is removably bonded to a second face (6, 6') of the substrate layer (2, 2'), on an opposite side to the first face (4, 4'), and extends beyond the substrate layer (2, 2') in at least a first direction, (15') along a first dimension, so as to aid alignment of the ultrasound transducer (8, 8') adjacent the carotid artery of the patient,
the method comprising:
using the alignment layer (10, 10') to align the ultrasound transducer (8, 8') over the carotid artery of the patient;
applying pressure to the substrate layer (2, 2') to bond the first adhesive surface (5, 5') to the skin of the patient; and
removing the alignment layer (10, 10') from the substrate layer (2, 2'), thereby exposing a region of the neck of the patient.

14. The method of claim 13, wherein the ultrasound transducer attachment system (1, 1') further comprises a backing cover (18, 18') covering the first adhesive surface (5, 5') of the substrate layer (2, 2'), the method further comprising removing the backing cover (18, 18') to expose the first adhesive surface (5, 5');
wherein the removable alignment layer (10, 10') comprises a first portion (12, 12'), removably bonded to the second face (6, 6') of the substrate layer (2, 2'), and a second portion (14, 14'), extending beyond the substrate layer (2, 2') in the first direction (15') and bonded to the backing cover (18, 18'), the method further comprising removing the backing cover (18, 18') to expose the first adhesive surface (5, 5'), such that, as the backing cover (18, 18') is removed, the removable alignment layer (10, 10') is opened out from a folded configuration.

15. The method of claim 13 or 14, wherein the method further comprises aligning the alignment layer (10, 10') such that the first direction (15') lies perpendicular to a median plane of the patient.

## Patentansprüche

1. Befestigungssystem für Ultraschallwandler (1, 1'), umfassend:
eine Substratschicht (2, 2'), die eine erste Fläche (4, 4') und eine zweite Fläche (6, 6') aufweist, wobei die zweite Fläche (6, 6') an der einer entgegengesetzten Seite zur ersten Fläche (4, 4') Fläche liegt, und die erste Fläche (4, 4') eine erste Haftoberfläche (5, 5') zum Kleben auf die Haut am Hals eines menschlichen oder tierischen Patienten umfasst;
einen Ultraschallwandler (8, 8'), der an der ersten Fläche (4, 4') der Substratschicht (2, 2') zum Platzieren angrenzend an eine Halsschlagader des Patienten fixiert ist; und
**dadurch gekennzeichnet, dass** es weiter eine entfernbare Ausrichtungsschicht (10, 10') umfasst, die entfernbar an die zweite Fläche (6, 6') der Substratschicht (2, 2') geklebt ist und sich über die Substratschicht (2, 2') hinaus in mindestens einer ersten Richtung (15') entlang einer ersten Dimension erstreckt, um die Ausrichtung des Ultraschallwandlers (8, 8') an die Halsschlagader des Patienten angrenzend zu unterstützen.

2. Befestigungssystem für Ultraschallwandler (1, 1') nach Anspruch 1, wobei die entfernbare Ausrichtungsschicht (10, 10') mindestens ein Ausrichtungsmerkmal (37') umfasst; wobei optional
das mindestens eine Ausrichtungsmerkmal (37') eine ausgeschnittene Kerbe, und/oder einen Vorsprung, und/oder eine sichtbare Oberflächenmarkierung umfasst; und/oder
wobei das mindestens eine Ausrichtungsmerkmal (37') eine Mitte der entfernbaren Ausrichtungsschicht (10, 10') in der ersten Dimension zum Ausrichten an eine Mitte des Halses des Patienten angrenzend angibt.

3. Befestigungssystem für Ultraschallwandler (1, 1') nach einem vorstehenden Anspruch, wobei sich die Substratschicht (2, 2') in der ersten Dimension zur Gänze an einer Seite einer Mittellinie der entfernbaren Ausrichtungsschicht (10, 10') befindet; und/oder
wobei die Substratschicht (2, 2') eine Ausdehnung in der ersten Richtung von mindestens 50 mm und von höchstens 125 mm aufweist.

4. Befestigungssystem für Ultraschallwandler (1, 1') nach einem vorstehenden Anspruch, wobei die entfernbare Ausrichtungsschicht (10, 10') in der ersten Dimension eine größte Ausdehnung aufweist, die mindestens doppelt so groß ist wie die größte Ausdehnung der Substratschicht (2, 2') in der ersten Dimension; und/oder
wobei die entfernbare Ausrichtungsschicht (10, 10') in der ersten Dimension länglich ist; und/oder
wobei die entfernbare Ausrichtungsschicht (10, 10') eine Ausdehnung in der ersten Dimension von mindestens 200 mm aufweist.

5. Befestigungssystem für Ultraschallwandler (1, 1') nach einem vorstehenden Anspruch, wobei die entfernbare Ausrichtungsschicht (10, 10') in einer zweiten Dimension senkrecht zur ersten Dimension eine Ausdehnung aufweist, das gleich einer Ausdehnung der Substratschicht (2, 2') in der zweiten Dimension ist.

6. Befestigungssystem für Ultraschallwandler (1, 1') nach einem vorstehenden Anspruch, wobei sich die entfernbare Ausrichtungsschicht (10, 10') in der ersten Richtung um mindestens 100 mm über die Substratschicht (2, 2') hinaus erstreckt.

7. Befestigungssystem für Ultraschallwandler (1, 1') nach einem vorstehenden Anspruch, wobei der Ultraschallwandler (8, 8') von einer Mittellinie der entfernbaren Ausrichtungsschicht (10, 10') in einer Richtung entgegengesetzt zur ersten Richtung versetzt ist; wobei optional
der Ultraschallwandler (8, 8') von der Mittellinie der entfernbaren Ausrichtungsschicht (10, 10') versetzt ist, sodass ein Mittelpunkt des Ultraschallwandlers (8, 8') in der ersten Dimension annähernd 50 mm von der Mittellinie der entfernbaren Ausrichtungsschicht (10, 10') entfernt liegt.

8. Befestigungssystem für Ultraschallwandler (1, 1') nach einem vorstehenden Anspruch, das weiter eine Trägerabdeckung (18, 18') umfasst, die die erste Haftoberfläche (5, 5') der Substratschicht (2, 2') abdeckt, wobei der Ultraschallwandler (8, 8') zwischen der Substratschicht (2, 2') und der Trägerabdeckung (18, 18') positioniert ist; wobei optional
die entfernbare Ausrichtungsschicht (10, 10') einen ersten Abschnitt (12, 12') umfasst, der mit einer ersten Haftfestigkeit an die zweite Fläche (6, 6') der Substratschicht (2, 2') geklebt ist, und einen zweiten Abschnitt (14, 14'), der sich in der ersten Richtung über die Substratschicht (2, 2') hinaus erstreckt und mit einer zweiten Haftfestigkeit, die größer ist als die erste Haftfestigkeit, an die Trägerabdeckung (18, 18') geklebt ist; und/oder
wobei die Trägerabdeckung (18, 18') eine Lasche (19, 19') umfasst, die sich in der ersten Dimension von der Substratschicht (2, 2') weg erstreckt, um das Entfernen der Trägerabdeckung (18, 18') von der Substratschicht (2, 2') zu unterstützen.

9. Befestigungssystem für Ultraschallwandler (1, 1') nach Anspruch 8, das weiter eine akustische Kopplungsschicht (16, 16') umfasst, die zwischen dem Ultraschallwandler (8, 8') und der Trägerabdeckung (18, 18') positioniert ist; wobei optional
die akustische Kopplungsschicht (16, 16') ein Ultraschallkopplungsgel, ein Hydrogel oder eine Silikonverbindung ist.

10. Befestigungssystem für Ultraschallwandler (1, 1') nach einem vorstehenden Anspruch, wobei die entfernbare Ausrichtungsschicht (10, 10') mit einer Haftfestigkeit an die Substratschicht (2, 2') geklebt ist, die geringer ist als eine Haftfestigkeit zwischen der ersten Haftoberfläche der Substratschicht und der Haut des Patienten, nachdem die erste Haftoberfläche an dem Patienten angelegt worden ist.

11. Befestigungssystem für Ultraschallwandler (1, 1') nach einem vorstehenden Anspruch, wobei die erste Haftoberfläche (5, 5') ein Hydrogel umfasst.

12. Befestigungssystem für Ultraschallwandler (1') nach einem vorstehenden Anspruch, wobei die Substratschicht (2') einen Schlitz (90') umfasst, der angeordnet ist, um ein Kabel (9') des Ultraschallwandlers (8') innerhalb des Schlitzes (90') aufzunehmen, und/oder es dem Kabel (9') zu ermöglichen, durch den Schlitz (90') durchzugehen; und/oder
wobei die entfernbare Ausrichtungsschicht (10') einen Schlitz (92') umfasst, der angeordnet ist, um ein Kabel (9') des Ultraschallwandlers (8') innerhalb des Schlitzes (92') aufzunehmen, und/oder es dem Kabel (9') zu ermöglichen, durch den Schlitz (92') durchzugehen.

13. Verfahren zum Befestigen eines Ultraschallwandlers (8, 8') an der Haut am Hals eines menschlichen oder tierischen Patienten, der angrenzend an eine Halsschlagader des Patienten ausgerichtet ist, wobei:
der Ultraschallwandler (8, 8') an einer ersten Fläche (4, 4') einer Substratschicht (2, 2') fixiert ist;
die erste Fläche (4, 4') eine erste Haftoberfläche (5, 5') zum Kleben an die Haut des Patienten umfasst; und
**dadurch gekennzeichnet, dass** eine entfernbare Ausrichtungsschicht (10, 10') entfernbar an eine zweite Fläche (6, 6') der Substratschicht (2, 2') an eine der ersten Fläche (4, 4') entgegengesetzten Seite geklebt ist und sich in mindestens einer ersten Richtung (15') entlang einer ersten Dimension über die Substratschicht (2, 2') hinaus erstreckt, um die Ausrichtung des Ultraschallwandlers (8, 8') an die Halsschlagader des Patienten angrenzend zu unterstützen, wobei das Verfahren umfasst:
Verwenden der Ausrichtungsschicht (10, 10'), um den Ultraschallwandler (8, 8') über der Halsschlagader des Patienten auszurichten;
Anlegen von Druck auf die Substratschicht (2, 2'), um die erste Haftoberfläche (5, 5') an die Haut des Patienten zu kleben; und
Entfernen der Ausrichtungsschicht (10, 10') von der Substratschicht (2, 2'), wodurch ein Bereich des Halses des Patienten freigelegt wird.

14. Verfahren nach Anspruch 13, wobei das Befestigungssystem für Ultraschallwandler (1,1') weiter eine Trägerabdeckung (18, 18') umfasst, die die erste Haftoberfläche (5, 5') der Substratschicht (2, 2') bedeckt, wobei das Verfahren weiter das Entfernen der Trägerabdeckung (18, 18') umfasst, um die erste Haftoberfläche (5, 5') freizulegen;
wobei die entfernbare Ausrichtungsschicht (10, 10') einen ersten Abschnitt (12, 12'), der entfernbar an die zweite Fläche (6, 6') der Substratschicht (2, 2') geklebt ist, und einen zweiten Abschnitt (14, 14') umfasst, der sich in der ersten Richtung (15') über die Substratschicht (2, 2') hinaus erstreckt und an die Trägerabdeckung (18, 18') geklebt ist, wobei das Verfahren weiter das Entfernen der Trägerabdeckung (18, 18') umfasst, um die erste Haftoberfläche (5, 5') freizulegen, sodass, wenn die Trägerabdeckung (18, 18') entfernt wird, die entfernbare Ausrichtungsschicht (10, 10') aus einer gefalteten Konfiguration heraus geöffnet wird.

15. Verfahren nach Anspruch 13 oder 14, wobei das Verfahren weiter das Ausrichten der Ausrichtungsschicht (10, 10') umfasst, sodass die erste Richtung (15') senkrecht zu einer Mittelebene des Patienten liegt.

## Revendications

1. Système de fixation de transducteur à ultrasons (1, 1'), comprenant :
une couche de substrat (2, 2'), présentant une première face (4, 4') et une seconde face (6, 6'), dans lequel la seconde face (6, 6') est sur un côté opposé à la première face (4, 4'), la première face (4, 4') comprenant une première surface adhésive (5, 5') pour une liaison à la peau du cou d'un patient humain ou animal ;
un transducteur à ultrasons (8, 8'), fixé à la première face (4, 4') de la couche de substrat (2, 2'), pour un placement adjacent à une artère carotide du patient ; et
**caractérisé en ce qu'**il comprend en outre une couche d'alignement amovible (10, 10'), liée de manière amovible à la seconde face (6, 6') de la couche de substrat (2, 2') et s'étendant au-delà de la couche de substrat (2, 2') dans au moins une première direction (15'), le long d'une première dimension, de manière à faciliter un alignement du transducteur à ultrasons (8, 8') adjacent à l'artère carotide du patient.

2. Système de fixation de transducteur à ultrasons (1,1') selon la revendication 1, dans lequel la couche d'alignement amovible (10, 10') comprend au moins une caractéristique d'alignement (37') ; facultativement
dans lequel la au moins une caractéristique d'alignement (37') comprend une encoche découpée et/ou une saillie et/ou une marque de surface visible ; et/ou
dans lequel la au moins une caractéristique d'alignement (37') indique un centre de la couche d'alignement amovible (10, 10') dans la première dimension pour un alignement adjacent à un centre du cou du patient.

3. Système de fixation de transducteur à ultrasons (1, 1') selon une quelconque revendication précédente, dans lequel la couche de substrat (2, 2') est située entièrement d'un côté d'une ligne centrale de la couche d'alignement amovible (10, 10') dans la première dimension ; et/ou
dans lequel la couche de substrat (2, 2') présente une étendue dans la première direction d'au moins 50 mm et d'au plus 125 mm.

4. Système de fixation de transducteur à ultrasons (1, 1') selon une quelconque revendication précédente, dans lequel la couche d'alignement amovible (10, 10') présente la plus grande étendue dans la première dimension qui fait au moins le double de la plus grande étendue de la couche de substrat (2, 2') dans la première dimension ; et/ou
dans lequel la couche d'alignement amovible (10, 10') est allongée dans la première dimension ; et/ou
dans lequel la couche d'alignement amovible (10, 10') présente une étendue dans la première dimension d'au moins 200 mm.

5. Système de fixation de transducteur à ultrasons (1, 1') selon une quelconque revendication précédente, dans lequel la couche d'alignement amovible (10, 10') présente une étendue dans une seconde dimension, perpendiculaire à la première dimension, qui est la même qu'une étendue de la couche de substrat (2, 2') dans la seconde dimension.

6. Système de fixation de transducteur à ultrasons (1, 1') selon une quelconque revendication précédente, dans lequel la couche d'alignement amovible (10, 10') s'étend d'au moins 100 mm au-delà de la couche de substrat (2, 2') dans la première direction.

7. Système de fixation de transducteur à ultrasons (1, 1') selon une quelconque revendication précédente, dans lequel le transducteur à ultrasons (8, 8') est décalé par rapport à une ligne centrale de la couche d'alignement amovible (10, 10') dans une direction opposée à la première direction ; facultativement
dans lequel le transducteur à ultrasons (8, 8') est décalé par rapport à la ligne centrale de la couche d'alignement amovible (10, 10') de telle sorte qu'un centre du transducteur à ultrasons (8, 8') soit approximativement à 50 mm de la ligne centrale de la couche d'alignement amovible (10, 10') dans la première dimension.

8. Système de fixation de transducteur à ultrasons (1, 1') selon une quelconque revendication précédente, comprenant en outre un revêtement de support (18, 18') recouvrant la première surface adhésive (5, 5') de la couche de substrat (2, 2'), dans lequel le transducteur à ultrasons (8, 8') est positionné entre la couche de substrat (2, 2') et le revêtement de support (18, 18') ; facultativement
dans lequel la couche d'alignement amovible (10, 10') comprend une première partie (12, 12'), liée à la seconde face (6, 6') de la couche de substrat (2, 2') avec une première force adhésive, et une seconde partie (14, 14'), s'étendant au-delà de la couche de substrat (2, 2') dans la première direction, et liée au revêtement de support (18, 18') avec une seconde force adhésive plus importante que la première force adhésive ; et/ou
dans lequel le revêtement de support (18, 18') comprend une languette (19, 19') s'étendant à l'opposé de la couche de substrat (2, 2') dans la première dimension, pour aider au retrait du revêtement de support (18, 18') de la couche de substrat (2, 2').

9. Système de fixation de transducteur à ultrasons (1, 1') selon la revendication 8, comprenant en outre une couche de couplage acoustique (16, 16') positionnée entre le transducteur à ultrasons (8, 8') et le revêtement de support (18, 18') ; facultativement
dans lequel la couche de couplage acoustique (16, 16') est un gel de couplage ultrasonore, un hydrogel ou un composé de silicone.

10. Système de fixation de transducteur à ultrasons (1, 1') selon une quelconque revendication précédente, dans lequel la couche d'alignement amovible (10, 10') est liée à la couche de substrat (2, 2') avec une force adhésive qui est inférieure à une force adhésive entre la première surface adhésive de la couche de substrat et la peau du patient, une fois que la première surface adhésive est appliquée sur le patient.

11. Système de fixation de transducteur à ultrasons (1, 1') selon une quelconque revendication précédente, dans lequel la première surface adhésive (5, 5') comprend un hydrogel.

12. Système de fixation de transducteur à ultrasons (1') selon une quelconque revendication précédente, dans lequel la couche de substrat (2') comprend une fente (90') agencée pour loger un câble (9') du transducteur à ultrasons (8') à l'intérieur de la fente (90'). et/ou pour permettre au câble (9') de passer à travers la fente (90') ; et/ou
dans lequel la couche d'alignement amovible (10') comprend une fente (92') agencée pour loger un câble (9') du transducteur à ultrasons (8') à l'intérieur de la fente (92') et/ou pour permettre au câble (9') de passer à travers la fente (92').

13. Procédé de fixation d'un transducteur à ultrasons (8, 8') sur la peau du cou d'un patient humain ou animal, aligné adjacent à une artère carotide du patient, dans lequel :
le transducteur à ultrasons (8, 8') est fixé sur une première face (4, 4') d'une couche de substrat (2, 2') ;
la première face (4, 4') comprend une première surface adhésive (5, 5') pour une liaison à la peau du patient ; et
**caractérisé en ce qu'**une couche d'alignement amovible (10, 10') est liée de manière amovible à une seconde face (6, 6') de la couche de substrat (2, 2'), sur un côté opposé à la première face (4, 4') et s'étend au-delà de la couche de substrat (2, 2') dans au moins une première direction (15'), le long d'une première dimension, de manière à faciliter un alignement du transducteur à ultrasons (8, 8') adjacent à l'artère carotide du patient, le procédé comprenant :
l'utilisation de la couche d'alignement (10, 10') pour aligner le transducteur à ultrasons (8, 8') au-dessus de l'artère carotide du patient ;
l'application d'une pression sur la couche de substrat (2, 2') pour lier la première surface adhésive (5, 5') à la peau du patient ; et
l'enlèvement de la couche d'alignement (10, 10') de la couche de substrat (2, 2'), ce qui permet ainsi d'exposer une région du cou du patient.

14. Procédé selon la revendication 13, dans lequel le système de fixation du transducteur à ultrasons (1, 1') comprend en outre un revêtement de support (18, 18') recouvrant la première surface adhésive (5, 5') de la couche de substrat (2, 2'), le procédé comprenant en outre l'enlèvement du revêtement de support (18, 18') pour exposer la première surface adhésive (5, 5') ;
dans lequel la couche d'alignement amovible (10, 10') comprend une première partie (12, 12'), liée de manière amovible à la seconde face (6, 6') de la couche de substrat (2, 2'), et une seconde partie (14, 14') s'étendant au-delà de la couche de substrat (2, 2') dans la première direction (15') et liée au revêtement de support (18, 18'), le procédé comprenant en outre l'enlèvement du revêtement de support (18, 18') pour exposer la première surface adhésive (5, 5'), de telle sorte que, lorsque le revêtement de support (18, 18') est retiré, la couche d'alignement amovible (10, 10') soit ouverte à partir d'une configuration pliée.

15. Procédé selon la revendication 13 ou 14, dans lequel le procédé comprend en outre l'alignement de la couche d'alignement (10, 10') de telle sorte que la première direction (15') soit perpendiculaire à un plan médian du patient.
